# EUROPÄISCHE PATENTSCHRIFT

(11) **EP 3 352 030 B1**
(45) Veröffentlichungstag und Bekanntmachung des Hinweises auf die Patenterteilung: **20.11.2019**
(21) Anmeldenummer: 17152779.9
(22) Anmeldetag: 24.01.2017
(51) Int. Cl.: G05B 19/042, G06F 3/01

(54) **TRAGBARE ERWEITERUNGSEINHEIT FÜR DIE BEDIENUNG EINES MEDIZINTECHNISCHEN GERÄTS UND VERFAHREN ZUM BETRIEB EINES MEDIZINTECHNISCHEN GERÄTS**
PORTABLE EXPANSION UNIT FOR THE OPERATION OF A MEDICAL DEVICE AND METHOD FOR OPERATING A MEDICAL DEVICE
UNITÉ D'EXTENSION PORTATIVE POUR L'UTILISATION D'UN APPAREIL MÉDICO-TECHNIQUE ET PROCÉDÉ DE FONCTIONNEMENT D'UN APPAREIL MÉDICO-TECHNIQUE

(43) Veröffentlichungstag der Anmeldung: 25.07.2018
(73) Patentinhaber: Siemens Healthcare GmbH, 91052 Erlangen (DE)
(72) Erfinder: Dirauf, Franz, 96250 Ebensfeld (DE); Kagermeier, Robert, 90427 Nürnberg (DE); Hofmann, Jörg, 91301 Forchheim (DE)

(56) Entgegenhaltungen:
- EP-A2- 2 034 463
- DE-A1-102008 060 117

## Beschreibung

### Gebiet der Erfindung

Die Erfindung betrifft eine tragbare Erweiterungseinheit, die eingerichtet ist, sicherheitsrelevante Funktionen von medizintechnischen Geräten zu steuern. Die Erfindung betrifft auch ein zugehöriges Verfahren zum Betrieb eines medizintechnischen Geräts.

### Hintergrund der Erfindung

Auch die Bedienung medizintechnischer Geräte soll zeitgemäß und daher intuitiv, übersichtlich und bequem gestaltet werden. Die Bedienoberflächen mobiler Tabletcomputer und Smartphones haben sich weitgehend etabliert und sind der Standard, der von den Benutzern nicht nur akzeptiert und verstanden wird, sondern mit Hilfe von Apps und HTML Darstellung bereits auf weite Bereiche des täglichen Lebens ausgedehnt wurde.

Diese Art einer Bedienoberfläche mit animierter graphischer Darstellung, Touch-Funktion und zusätzlicher Sensorik, wie Neigungs- und Beschleunigungssensoren, Kameras etc., eignet sich auch hervorragend für die Bedienung von medizintechnischen Geräten.

Allerdings erfüllen Consumer-Geräte nicht die Anforderungen, die für sicherheitsrelevante Funktionen der Medizintechnik, wie Bewegungssteuerung, Strahlungsfreigabe etc., zu erfüllen sind. Diese Funktionen müssen so gesteuert werden, dass sie nicht durch einen einfachen Hardware- oder Softwarefehler unbeabsichtigt freigegeben werden und damit Schaden anrichten können ("Erstfehlersicherheit").

Bisher werden ausschließlich speziell entwickelte Bediengeräte verwendet, die oft nur einfache und wenig intuitive Bedienfunktionen ermöglichen und aufgrund ihrer geringen Stückzahlen vergleichsweise teuer sind.

In der Offenlegungsschrift EP 2 034 463 A2 wird ein Verfahren und eine Vorrichtung zur drahtlosen Übertragung von Signalen zwischen einem mobilen Bedienteil und einem Basisteil eines medizinischen Behandlungsgeräts offenbart. Dabei werden die zu übertragenden Signale kategorisiert, nämlich in sicherheitsrelevante Steuersignale und unkritische Kommunikationssignale. Lediglich die sicherheitsrelevanten Steuersignale werden als codierte Signale übertragen und auf Übertragungsfehlersicherheit überprüft.

Die Offenlegungsschrift DE 10 2008 0601 117 A1 offenbart eine Maschine mit einer Steuerungseinrichtung zum Steuern einer Funktion dieser Maschine und mit einem Bediengerät zum Bedienen der Maschine. Das Bediengerät weist ein erstes Betätigungselement auf, wobei durch eine Betätigung dieses Betätigungselements die Funktion der Maschine auslösbar ist und wobei das Betätigungselement über eine erste Verbindung mit der Steuerungseinrichtung in Kommunikationsverbindung steht. Die erste Verbindung ist eine nicht sicherheitsgerechte Verbindung und das Bediengerät weist daher ein zweites Betätigungselement auf, wobei dieses zweite Betätigungselement über eine sicherheitsgerechte zweite Verbindung mit der Steuerungseinrichtung in Kommunikationsverbindung steh und wobei zum Auslösen der Funktion der Maschine ein Zusammenwirken des ersten und des zweiten Betätigungselements erforderlich ist.

### Zusammenfassung der Erfindung

Es ist Aufgabe der Erfindung, handelsübliche und sehr kostengünstige mobile, tragbare Consumer-Bediengeräte, wie Tabletcomputer und Smartphones, für die Bedienung von medizintechnischen Geräten zu ertüchtigen.

Gemäß der Erfindung wird die gestellte Aufgabe mit der Erweiterungseinheit und dem Verfahren zum Betrieb eines medizintechnischen Geräts der unabhängigen Patentansprüche gelöst. Vorteilhafte Weiterbildungen sind in den abhängigen Ansprüchen angegeben.

Erfindungsgemäß wird die Bedienung sicherheitsrelevanter Funktionen an eine externe Zusatzeinheit (= Erweiterungseinheit) ausgelagert, die entsprechend den zutreffenden Regeln und Standards qualifiziert ist (Stichwort "Erstfehlersicherheit"). Das mobile Bediengerät (= tragbare Bedieneinheit) selbst unterliegt somit nicht den sicherheitstechnischen Anforderungen. Die üblicherweise sehr kurzen Modellzyklen bei derartigen Consumer- Bediengeräten stellen damit bezüglich der Qualifikation und Zulassung kein Problem mehr dar.

Erfindungsgemäß wird eine Erweiterungseinheit für die drahtlose Ausführung von sicherheitsrelevanten Funktionen bei der Bedienung medizintechnischer Geräte angegeben. Die Erweiterungseinheit kann mit handelsüblichen flachen, tragbaren Computern ("Flachrechner"), wie Tabletcomputer, Smartphones, etc., kombiniert werden. Dabei wird die Erweiterungseinheit mittels einer variablen, auf die Baugröße des tragbaren Bediengeräts einstellbaren Haltevorrichtung (zum Beispiel mit Hilfe eines Hebels klemmbar oder mit einem Federzug ausgestattet) oder einer Halteschale mechanisch mit der tragbaren Bedieneinheit verbunden.

Die Erweiterungseinheit ist über eine Funkverbindung oder auch durch eine Kabelverbindung mit einem Empfängermodul des medizintechnischen Geräts verbunden. Über die digitale Kommunikation, z. B. mit Bluetooth oder WLAN, wird ein gesichertes Protokoll mit einer Erkennung von Übertragungsfehlern und Zeitüberschreitungen übertragen.

Die Erfindung beansprucht eine tragbare Erweiterungseinheit, die eingerichtet ist, sicherheitsrelevante Funktionen von medizintechnischen Geräten zu steuern, beispielsweise auszulösen oder freizugeben. Sie weist dazu auf:
- eine auf die Größe einer flach ausgebildeten, tragbaren Bedieneinheit des medizintechnischen Geräts anpassbare Haltevorrichtung, die ausgebildet ist, die Erweiterungseinheit lösbar mit der Bedieneinheit mechanisch zu verbinden, und
- ein erstes Kommunikationsmodul, das ausgebildet ist, eine sicherheitsgerichtete Datenverbindung mit dem medizintechnischen Gerät herzustellen.

Der Vorteil der Erfindung besteht in der Kombination eines mobilen, tragbaren Consumer Bediengeräts, wie Tabletcomputer oder Smartphone, das keine besonderen sicherheitstechnischen Anforderungen erfüllt, mit einer Erweiterungseinheit, die eine Steuerung sicherheitsrelevanter Funktionen mit Hilfe eines sicherheitsgerichteten Funkprotokolls ermöglicht.

In einer Weiterbildung kann die Haltevorrichtung ausgebildet sein, die Erweiterungseinheit von der Unterseite der Bedieneinheit her auf die Bedieneinheit zu klemmen.

In einer weiteren Ausgestaltung weist die Haltevorrichtung einen verschiebbaren, in der Haltevorrichtung stufenlos fixierbaren Hebel auf, der ausgebildet ist, die Bedieneinheit in die Erweiterungseinheit zu klemmen.

In einer optionalen Ausführungsform weist die Haltevorrichtung einen verschiebbaren Hebel mit Federzug auf, der ausgebildet ist, die Bedieneinheit in die Erweiterungseinheit zu klemmen.

In einer weiteren Ausprägung kann die Unterseite der Erweiterungseinheit derart flach ausgebildet sein, dass die mit der Bedieneinheit verbundene Erweiterungseinheit auf einer ebenen Fläche pultartig abstellbar ist.

In einer Weiterbildung kann das erste Kommunikationsmodul ausgebildet sein, eine Bluetooth- oder WLAN- Verbindung aufzubauen.

Die Erweiterungseinheit kann in einer weiteren Ausgestaltung ein zweites Kommunikationsmodul aufweisen, das ausgebildet ist, mit der Bedieneinheit Daten auszutauschen.

Des Weiteren kann das zweite Kommunikationsmodul über NFC oder USB mit der Bedieneinheit verbunden sein.

In einer Weiterbildung kann die Erweiterungseinheit ein einem Nutzer im Betrieb zugewandtes Bedienfeld aufweisen, das ausgebildet ist, bei einer mechanischen Verbindung der Erweiterungseinheit mit der Bedieneinheit über die Bedieneinheit vor zu kragen.

Das Bedienfeld kann in einer weiteren Ausgestaltung der Erfindung einen Touchscreen oder eine Folientastatur aufweisen.

In einer bevorzugten Ausgestaltung kann die Bedieneinheit ein Tabletcomputer oder ein Smartphone sein.

In einer Weiterbildung kann mindestens einen Taster an der Haltevorrichtung angeordnet sein, der von einem Benutzer der Erweiterungseinheit mit der die Erweiterungseinheit haltenden Hand ausgelöst werden kann.

Die Erfindung beansprucht auch ein Verfahren zum Betrieb eines medizintechnischen Geräts, wobei sicherheitsrelevante Funktionen der Bedienung des medizintechnischen Geräts durch eine erfindungsgemäße Erweiterungseinheit ausgelöst werden.

Weitere Besonderheiten und Vorteile der Erfindung werden aus den nachfolgenden Erläuterungen eines Ausführungsbeispiels anhand von schematischen Zeichnungen ersichtlich.

Es zeigen:
- Fig. 1:: eine Erweiterungseinheit an einem Tabletcomputer von oben,
- Fig. 2:: eine Erweiterungseinheit an einem Tabletcomputer von unten,
- Fig. 3:: eine Detailansicht der Haltevorrichtung einer Erweiterungseinheit und
- Fig. 4:: ein Blockschaltbild einer Anordnung mit einer Erweiterungseinheit und einem medizintechnischen Gerät.

### Detaillierte Beschreibung eines Ausführungsbeispiels

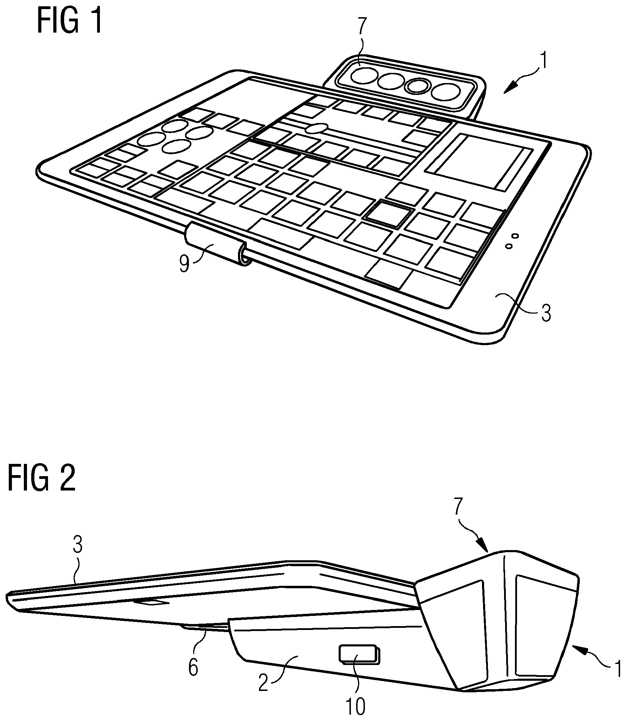

**Fig. 1** zeigt eine tragbare Erweiterungseinheit 1, die an einer tragbaren, flachen Bedieneinheit 3 (beispielsweise ein handelsüblicher Tabletcomputer) befestigt ist. Die Erweiterungseinheit 1 umschließt klammerartig die Bedieneinheit 3 von ihrer Rückseite her. Dazu besitzt sie eine Haltevorrichtung, von der das krallenförmige Ende 9 zu erkennen ist. Dieses umschließt den Rand der Bedieneinheit 3. Mit Hilfe der Bedieneinheit 3 werden alle Paramater eines medizintechnischen Geräts eingestellt und nicht-sicherheitsrelevante Funktionen gesteuert.

Zu erkennen ist auch das Bedienfeld 7 der Erweiterungseinheit 1, mit Hilfe dessen sicherheitsrelevante Funktionen eines medizintechnischen Geräts (beispielsweise eine Röntgenstrahlauslösung) gesteuert werden können. Das Bedienfeld 7 kann beispielsweise als Touchscreen oder als Folientastatur ausgebildet sein. Es kragt über die Bedieneinheit 3 vor, so dass es gut sichtbar und bedienbar ist.

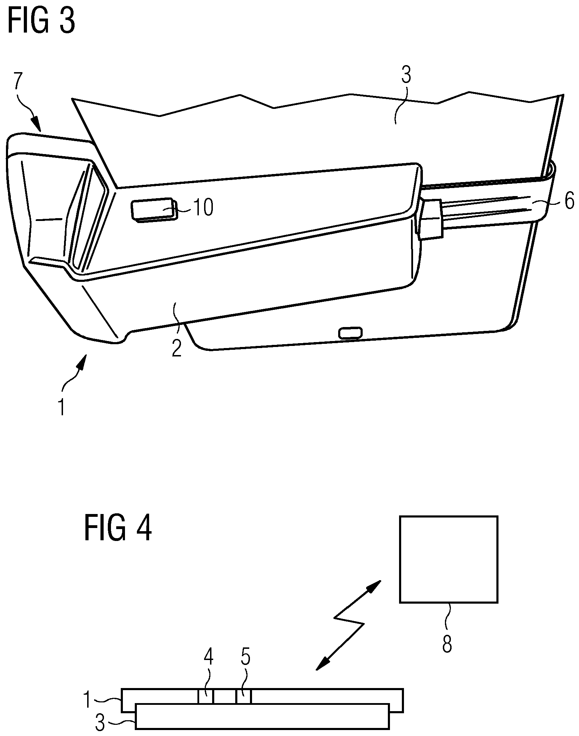

**Fig. 2** und **Fig. 3** zeigen die Anordnung der **Fig. 1** von unten. Die Erweiterungseinheit 1 ist mit Hilfe der Haltevorrichtung 2 von der Rückseite der Bedieneinheit 3 an der Bedieneinheit 3 lösbar befestigt. Dazu weist die Haltevorrichtung 2 einen Hebel 6 auf, der als Rasthebel oder als Federzug ausgebildet sein kann. Dadurch wird die Erweiterungseinheit 1 an die Bedieneinheit 3 geklemmt.

Die Unterseite des Gehäuses der Haltevorrichtung 2 ist eben und angeschrägt, so dass die Bedieneinheit 3 auf einer ebenen Fläche sicher und verwackelfrei abgestellt werden kann. Die Erweiterungseinheit 1 wirkt somit als Pult für die Bedieneinheit 3.

An der Haltevorrichtung 2 können noch zusätzlich ein Taster 10 angeordnet sein, die eine einhändige Betätigung mit der Hand, die Erweiterungseinheit hält, erlaubt.

**Fig. 4** zeigt ein Blockschaltbild einer Anordnung mit einer Erweiterungseinheit 1 und einem medizintechnischen Gerät 8. Die Erweiterungseinheit 1 weist ein erstes Kommunikationsmodul 4 und ein zweites Kommunikationsmodul 5 auf. Mit dem ersten Kommunikationsmodul 4 wird eine sichere Datenverbindung, beispielsweise über Bluetooth oder WLAN, zum medizintechnischen Gerät 8 aufgebaut. Mit dem zweiten Kommunikationsmodul 5 kann mit der Bedieneinheit 3 über NFC oder USB kommuniziert werden.

Die Erweiterungseinheit 1 hat folgende Eigenschaften und Funktionen:
- Sie ermöglicht gemäß zu erfüllender regulatorischer Vorschriften auch sicherheitsrelevante Funktionen, wie Bewegungen oder Strahlungsfreigabe von medizintechnischen Geräten 8, per Tastendruck drahtlos zu steuern.
- Bei Bedarf kann auch eine Not-Stopp Funktion realisiert werden, wobei der Not-Stopp-Schalter über eine farbige Anzeige dem Benutzer signalisiert, ob er momentan aktiv oder inaktiv ist, d.h. ob aktuell eine sicherheitsgerichtete Kommunikationsverbindung besteht oder nicht.
- Die Zuordnung zwischen einer Bedieneinheit 3, wie Tabletcomputer oder Smartphone, zu einer Erweiterungseinheit 1 kann über die Vergabe von ID-Codes fest zugordnet werden. Alternativ kann die Zuordnung auch automatisch über eine Nahfeld-Funkverbindung (z.B. NFC) oder über eine kabelgebundene Kommunikation (z.B. USB oder Audio-Interface) erfolgen.
- Um die Datensicherheit wichtiger Einstellparameter zu gewährleisten, kann die Erweiterungseinheit 1 die Konsistenz dieser Parameter überprüfen, bevor eine sicherheitsrelevante Funktion, wie Bewegung oder Strahlung, durch einen Tastendruck auf dem Bedienfeld 7 freigegeben werden kann. Zu diesem Zweck erhält die Erweiterungseinheit 1 die gewünschten Einstellparameter von der Bedieneinheit 3, z.B. über eine Nahfeld-Funkverbindung oder eine kabelgebundene Kommunikation. Vom medizintechnischen Gerät 8 erhält die Erweiterungseinheit 1 die tatsächlich eingestellten Parameter über die sichere Funkverbindung. Nur wenn die gewünschten und die am medizinischen Gerät 8 eingestellten Parameter übereinstimmen, wird die jeweilige Funktion frei gegeben.
- Die Erweiterungseinheit 1 kann durch geeignete Codierung der drahtlosen Kommunikation (mit Hilfe des ersten Kommunikationsmoduls 4) und die Vergabe von ID-Codes an einem oder mehreren medizintechnischen Geräten 8 registriert werden, um diese zu bedienen.
- Sofern die Erweiterungseinheit 1 an mehreren Geräten 8 registriert ist, kann der Benutzer auf der Bedienoberfläche der Bedieneinheit 3 auswählen, welches registrierte medizintechnische Gerät 8 er bedienen möchte. Die Bedienoberfläche des Bedienfelds 7 passt sich dann selbsttätig an die Erfordernisse des jeweiligen Geräts 8 an. Alternativ kann die Auswahl des zu bedienenden Geräts 8 über drahtlose Kommunikation mittels Nahfeld-Funkverbindung (z. B. NFC) erfolgen.
- Die Formgebung der Erweiterungseinheit 1 ist so ausgelegt, dass es zusammen mit der Bedieneinheit 3 oder sicher von unten mit den Fingern einer Hand gehalten und mit der anderen Hand einfach bedient werden kann.
- Aufgrund der abgeschrägten Form der Erweiterungseinheit 1 wird die Bedieneinheit 3 beim Ablegen auf einem Tisch in eine Pult-Position gebracht, so dass die Bedienung sehr ergonomisch und bequem erfolgen kann.

Obwohl die Erfindung im Detail durch die Ausführungsbeispiele näher illustriert und beschrieben wurde, ist die Erfindung durch die offenbarten Beispiele nicht eingeschränkt und andere Variationen können vom Fachmann daraus abgeleitet werden, ohne den Schutzumfang der Erfindung zu verlassen.

### Bezugszeichenliste

- 1: Erweiterungseinheit
- 2: Haltevorrichtung
- 3: Bedieneinheit
- 4: erstes Kommunikationsmodul
- 5: zweites Kommunikationsmodul
- 6: Hebel der Haltevorrichtung 2
- 7: Bedienfeld der Erweiterungseinheit 1
- 8: medizintechnisches Gerät
- 9: krallenförmiges Ende des Hebels 6
- 10: Taster

## Patentansprüche

1. Tragbare Erweiterungseinheit (1), die eingerichtet ist, sicherheitsrelevante Funktionen von medizintechnischen Geräten (8) zu steuern, aufweisend:
- eine auf die Größe einer flach ausgebildeten, tragbaren Bedieneinheit (3) des medizintechnischen Geräts (8) anpassbare Haltevorrichtung (2), die ausgebildet ist, die Erweiterungseinheit (1) lösbar mit der tragbaren Bedieneinheit (3) mechanisch zu verbinden, und
- ein erstes Kommunikationsmodul (4), das ausgebildet ist, eine sicherheitsgerichtete Datenverbindung mit dem medizintechnischen Gerät (8) herzustellen.

2. Erweiterungseinheit (1) nach Anspruch 1,
**dadurch gekennzeichnet,**
**dass** die Haltevorrichtung (2) ausgebildet ist, die Erweiterungseinheit (1) von der Unterseite der Bedieneinheit (3) her auf die tragbaren Bedieneinheit (3) zu klemmen.

3. Erweiterungseinheit (1) nach Anspruch 2,
**gekennzeichnet durch:**
- einen verschiebbaren, in der Haltevorrichtung (2) stufenlos fixierbaren Hebel (6) der Haltevorrichtung (2), der ausgebildet ist, die tragbare Bedieneinheit (3) in der Erweiterungseinheit (1) zu fixieren.

4. Erweiterungseinheit (1) nach Anspruch 2,
**gekennzeichnet durch:**
- einen verschiebbaren Hebel (6) mit einem Federzug der Haltevorrichtung (2), der ausgebildet ist, die tragbare Bedieneinheit (3) in der Erweiterungseinheit (1) zu fixieren.

5. Erweiterungseinheit (1) nach einem der vorehrgehenden Ansprüche,
**dadurch gekennzeichnet,**
**dass** die Unterseite der Erweiterungseinheit (1) derart flach ausgebildet ist, dass die mit der tragbaren Bedieneinheit (3) verbundene Erweiterungseinheit (1) auf einer ebenen Fläche pultartig abstellbar ist.

6. Erweiterungseinheit (1) nach einem der vorhergehenden Ansprüche,
**dadurch gekennzeichnet,**
**dass** das erste Kommunikationsmodul (4) ausgebildet ist, eine Bluetooth- oder WLAN- Verbindung aufzubauen.

7. Erweiterungseinheit (1) nach einem der vorhergehenden Ansprüche,
**gekennzeichnet durch:**
- ein zweites Kommunikationsmodul (5), das ausgebildet ist, mit der tragbaren Bedieneinheit (3) Daten auszutauschen.

8. Erweiterungseinheit (1) nach Anspruch 7,
**dadurch gekennzeichnet,**
**dass** das zweite Kommunikationsmodul (5) über NFC oder USB mit der tragbaren Bedieneinheit (3) verbunden ist.

9. Erweiterungseinheit (1) nach einem der vorhergehenden Ansprüche,
**gekennzeichnet durch:**
- ein einem Nutzer im Betrieb zugewandtes Bedienfeld (7), das ausgebildet ist, nach einer mechanischen Verbindung der Erweiterungseinheit (1) mit der tragbaren Bedieneinheit (3) über die Bedieneinheit (3) vor zu kragen.

10. Erweiterungseinheit (1) nach Anspruch 9,
**dadurch gekennzeichnet,**
**dass** das Bedienfeld (7) einen Touchscreen aufweist.

11. Erweiterungseinheit (1) nach einem der vorhergehenden Ansprüche,
**dadurch gekennzeichnet,**
**dass** die Bedieneinheit (3) ein Tabletcomputer oder ein Smartphone ist.

12. Erweiterungseinheit (1) nach einem der vorhergehenden Ansprüche,
**gekennzeichnet durch:**
- mindestens einen Taster (10) an der Haltevorrichtung (2), der ausgebildet ist, von einem Benutzer der Erweiterungseinheit (1) mit der die Erweiterungseinheit (1) haltenden Hand auszulösen.

13. Verfahren zum Betrieb eines medizintechnischen Geräts (8), wobei sicherheitsrelevante Funktionen der Bedienung des medizintechnischen Geräts (8) durch eine tragbare Erweiterungseinheit (1) nach einem der vorhergehenden Ansprüche ausgelöst werden.

## Claims

1. Portable expansion unit (1), which is configured to control safety-relevant functions of medical devices (8), having:
- a retaining device (2) which is adjustable to the size of a flat, portable operating unit (3) of the medical device (8), said retaining device being configured to mechanically connect the expansion unit (1) detachably with the portable operating unit (3), and
- a first communication module (4), which is configured to establish a safety-related data connection with the medical device (8).

2. Expansion unit (1) according to claim 1,
**characterised in that,**
the retaining device (2) is configured to clamp the expansion unit (1) from the underside of the operating unit (3) to the portable operating unit (3).

3. Expansion unit (1) according to claim 2,
**characterised in:**
- a displaceable lever (6) of the retaining device (2) which can be continuously fixed in the retaining device (2) and which is configured to fix the portable operating unit (3) in the expansion unit (1).

4. Expansion unit (1) according to claim 2,
**characterised in:**
- a displaceable lever (6) with a spring balancer of the retaining device (2), the lever being configured to fix the portable operating unit (3) in the expansion unit (1).

5. Expansion unit (1) according to one of the preceding claims,
**characterised in that,**
the underside of the expansion unit (1) is configured to be flat such that the expansion unit (1) connected to the portable operating unit (3) is placable on a flat surface in the manner of a desk.

6. Expansion unit (1) according to one of the preceding claims,
**characterised in that,**
the first communication module (4) is configured to set up a Bluetooth or WLAN connection.

7. Expansion unit (1) according to one of the preceding claims,
**characterised in:**
- a second communication module (5) which is configured to exchange data with the portable operating unit (3).

8. Expansion unit (1) according to claim 7,
**characterised in that,**
the second communication module (5) is connected via NFC or USB to the portable operating unit (3).

9. Expansion unit (1) according to one of the preceding claims,
**characterised in:**
- an operating field (7) facing a user during operation, which is embodied to project beyond the operating unit (3) following a mechanical connection of the expansion unit (1) with the portable operating unit (3).

10. Expansion unit (1) according to claim 9,
**characterised in that,**
the operating field (7) has a touchscreen.

11. Expansion unit (1) according to one of the preceding claims,
**characterised in that,**
the operating unit (3) is a tablet computer or a smart phone.

12. Expansion unit (1) according to one of the preceding claims,
**characterised in:**
- at least one button (10) on the retaining device (2), which is configured to be triggered with the hand of a user of the expansion unit (1), the hand holding the expansion unit (1) .

13. Method for operating a medical device (8), wherein safety-relevant functions of the operation of the medical device (8) are triggered by a portable expansion unit (1) according to one of the preceding claims.

## Revendications

1. Unité (1) d'extension portative, conçue pour commander des fonctions, pertinentes du point de vue de la sécurité, d'appareils (8) de la technique médicale, comportant :
- un dispositif (2) de retenue, qui peut être adapté à la dimension d'une unité (3) de commande, portative et constituée de manière plate, de l'appareil (8) de la technique médicale et qui est constitué pour assembler mécaniquement l'unité (1) d'extension, de manière détachable, à l'unité (3) de commande portative, et
- un premier module (4) de communication, constitué pour ménager une liaison de données sécurisée avec l'appareil (8) de la technique médicale.

2. Unité (1) d'extension suivant la revendication 1,
**caractérisée**
**en ce que** le dispositif (2) de retenue est constitué pour serrer l'unité (1) d'extension depuis le côté inférieur de l'unité (3) de commande sur l'unité (3) de commande portative.

3. Unité (1) d'extension suivant la revendication 2,
**caractérisée par** :
- un levier (6) pouvant se déplacer et pouvant être immobilisé sans palier dans le dispositif (2) de retenue, du dispositif (2) de retenue, levier qui est constitué pour immobiliser l'unité (3) de commande portative dans l'unité (1) d'extension.

4. Unité (1) d'extension suivant la revendication 2,
**caractérisée par** :
- un levier (6) pouvant être déplacé et ayant une traction de ressort du dispositif (2) de retenue, levier constitué pour immobiliser l'unité (3) de commande portative dans l'unité (1) d'extension.

5. Unité (1) d'extension suivant l'une des revendications précédentes,
**caractérisée**
**en ce que** le côté inférieur de l'unité (1) d'extension est constitué de manière plate de manière à pouvoir mettre, à la manière d'un pupitre, sur une surface plane, l'unité (1) d'extension reliée à l'unité (3) de commande portative.

6. Unité (1) d'extension suivant l'une des revendications précédentes,
**caractérisée**
**en ce que** le premier module (4) de communication est constitué pour établir une liaison Bluetooth ou WLAN.

7. Unité (1) d'extension suivant l'une des revendications précédentes,
**caractérisée par** :
- un deuxième module (5) de communication, constitué pour échanger des données avec l'unité (3) de commande portative.

8. Unité (1) d'extension suivant la revendication 7,
**caractérisée**
**en ce que** le deuxième module (5) de communication est relié à l'unité (3) de commande portative par NFC ou USB.

9. Unité (1) d'extension suivant l'une des revendications précédentes,
**caractérisée par** :
- un champ (7) de commande, tourné en fonctionnement vers l'utilisateur et constitué pour, après un assemblage mécanique de l'unité (1) d'extension à l'unité (3) de commande portative, être mise en console par l'unité (3) de commande.

10. Unité (1) d'extension suivant la revendication 9,
**caractérisée**
**en ce que** le champ (7) de commande a un écran tactile.

11. Unité (1) d'extension suivant l'une des revendications précédentes,
**caractérisée**
**en ce que** l'unité (3) de commande est un ordinateur à tablette ou un smartphone.

12. Unité (1) d'extension suivant l'une des revendications précédentes,
**caractérisée par** :
- au moins une touche (10) sur le dispositif (2) de retenue, laquelle est constituée pour se déclencher par un utilisateur de l'unité (1) d'extension de la main tenant l'unité (1) d'extension.

13. Procédé pour faire fonctionner un appareil (8) de la technique médicale, des fonctions, pertinentes pour la sécurité de la commande de l'appareil (8) de la technique médicale, étant déclenchées par une unité (1) d'extension portative suivant l'une des revendications précédentes.
